(19) Europäisches Patentamt
European Patent Office
Office européen des brevets

(11) Veröffentlichungsnummer: **0 038 970**
**B1**

(12) # EUROPÄISCHE PATENTSCHRIFT

(45) Veröffentlichungstag der Patentschrift: **06.04.83**

(51) Int. Cl.³: **C 07 C 21/18**, C 07 C 17/38, B 01 D 53/14, C 08 F 6/10

(21) Anmeldenummer: **81102686.3**

(22) Anmeldetag: **09.04.81**

(54) **Verfahren zur Abtrennung von Tetrafluorethylen aus Gasgemischen, die neben Tetrafluorethylen insbesondere Stickstoff oder Kohlenmonoxid enthalten.**

(30) Priorität: **19.04.80 DE 3015092**

(43) Veröffentlichungstag der Anmeldung: **04.11.81 Patentblatt 81/44**

(45) Bekanntmachung des Hinweises auf die Patenterteilung: **06.04.83 Patentblatt 83/14**

(84) Benannte Vertragsstaaten: **BE DE FR GB IT NL**

(56) Entgegenhaltungen:
**DE-A-2 711 243**
**Chemical Abstracts Band 79, Nr. 3, 1973 Columbus, Ohio, USA: S. YOSHIDA et al. "Tetrafluoroethylene recovery", Seite 401, Spalte 1, Abstract Nr. 18050g.**
**Chemical Abstracts Band 82, Nr. 19, 1975 Columbus, Ohio, USA: H. AISO et al. "Removal of trace oxygen in tetrafluoroethylene", Seite 507, Spalte 2, Abstract Nr. 124701s.**

(73) Patentinhaber: **HOECHST AKTIENGESELLSCHAFT, Postfach 80 03 20, D-6230 Frankfurt/Main 80 (DE)**

(72) Erfinder: **Burgstaller, Gottfried, Ludwigshafener Strasse 3, D-8261 Burgkirchen/Alz (DE)**
Erfinder: **Freudenreich, Reinhold, Hochriesstrasse 15, D-8261 Burgkirchen/Alz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

Verfahren zur Abtrennung von Tetrafluorethylen aus Gasgemischen, die neben Tetrafluorethylen insbesondere Stickstoff oder Kohlenmonoxid enthalten.

Die Erfindung bezieht sich auf ein Verfahren zur Abtrennung von Tetrafluorethylen aus einem Gasgemisch, das bei der Verflüssigung der Pyrolyse-Produkte des Difluormonochlormethans bei Drücken von 1 bis 5 bar als nicht-verflüssigbarer Anteil, bei der Tetrafluorethylen-Herstellung oder -Polymerisation als Spülgas anfällt oder durch Vermischen von nicht-verflüssigbaren Anteilen und Spülgasen entsteht und das neben Tetrafluorethylen Kohlenmonoxid, Stickstoff oder Gemische dieser beiden Gase, ferner gegebenenfalls kleinere Anteile von Difluormonochlormethan sowie von dessen nicht-verflüssigbaren Pyrolyse-Produkten enthält.

Gasgemische der eingangs beschriebenen Art können an verschiedenen Stellen von Anlagen für die Herstellung von Tetrafluorethylen und dessen Weiterverarbeitung, beispielsweise in Polymerisationsprozessen, anfallen.

Kohlenomonoxid entsteht als gasförmiges Nebenprodukt, wenn bei der Pyrolyse von Difluormonochlormethan zu Tetrafluorethylen in Gegenwart von Wasserdampf gearbeitet wird. Auch wenn Stickstoff als inertes Verdünnungsmedium bei der Pyrolyse von Difluormonochlormethan zu Tetrafluorethylen im allgmeinen vermieden wird, fallen dennoch Gemische von Tetrafluorethylen und Stickstoff häufig an, wenn Reaktionsgefässe für die Polymerisation von Tetrafluorethylen zur Entfernung von Luftsauerstoff mit Stickstoff und anschliessend mit Tetrafluorethylen gespült werden. Ein Anfall von Stickstoff entsteht auch dann, wenn beim Anfahren der zur Tetrafluorethylen-Herstellung benutzten Pyrolyseöfen diese zuvor mit Stickstoff gespült werden.

Da Tetrafluorethylen eine ausserordentlich wertvolle Substanz darstellt und bezüglich der Umweltbelastung nicht in die Atmosphäre abgeführt werden soll, ist seine Wiedergewinnung - auch wenn es nur in kleinen Anteilen im Gasgemisch auftritt - erforderlich. Daraus resultiert das Problem, Gemische von Tetrafluorethylen und Kohlenmonoxid oder von Tetrafluorethylen und Stickstoff zu trennen.

Üblicherweise werden die Pyrolysegase des Difluormonochlormethans nach Auswaschen des Nebenprodukts Chlorwasserstoff vor ihrer weiteren Aufarbeitung in einem Gasometer zwischengespeichert, und diesem Gasometer werden auch die in der gesamten Tetrafluorethylen-Anlage anfallenden Spülgase zugeführt, so dass das dann der weiteren Aufarbeitung zugeführte Gasgemisch sowohl Kohlenmonoxid als auch Stickstoff enthält, daneben die Produkte der Pyrolyse, insbesondere das erwünschte Tetrafluorethylen, ferner das bei der Pyrolyse nicht-umgesetzte Difluormonochlormethan sowie kleinere Anteile von Kohlendioxid. Aus den Spülgasen können ausserdem geringe Mengen von Sauerstoff in das Gasgemisch gelangen.

Ein solches Gasgemisch wird zur Gewinnung des erwünschten Produkts Tetrafluorethylen aus dem Spektrum der bei der Pyrolyse anfallenden Fluorchlorkohlenwasserstoffe, Fluorkohlenwasserstoffe, Fluorchlorkohlenstoffe und Fluorkohlenstoffe im allgemeinen einer fraktionierten Destillation unterworfen, wozu zunächst die Verflüssigung des Gasgemisches erforderlich ist.

Da Tetrafluorethylen bei Drücken über etwa 6 bar zum explosiven Selbstzerfall neigt, ist eine Verflüssigung unter solchen stärker erhöhten Drücken nicht zweckmässig, und daher wird die Verflüssigung bei Drücken von 1 bis 5 bar, insbesondere von 1 bis 1,5 bar, vorgezogen, wobei sich, je nach der Zusammensetzung des Pyrolysegases, eine Temperatur von -10 bis -70°C, insbesondere von -60 bis -70°C, einstellt. Eine Verflüssigung bei noch wesentlich niedrigeren Temperaturen, mit der die Kondensation aller gasförmigen Bestandteile erreicht werden könnte, ist aus wirtschaftlichen Überlegungen nicht zweckmässig.

Somit resultiert aus der Verflüssigung solcher Gasgemische ein Teilstrom der verflüssigten Anteile, der der weiteren Aufarbeitung durch fraktionierte Destillation zugeführt wird, und ein Teilstrom von unter diesen Verflüssigungsbedingungen nicht-verflüssigbaren Anteilen, der - neben den tiefsiedenden Komponenten, wie vor allem Stickstoff und/oder Kohlenmonoxid, nicht umgesetztes Difluormonochlormethan sowie ferner niedriger als Tetrafluorethylen siedende Pyrolyseprodukte - insbesondere das partialdruckmässig mitgeführte Tetrafluorethylen, gegebenenfalls in beträchtlichen Anteilen, enthält. Die Zusammensetzung dieses nicht-verflüssigbaren Teilstroms ist abhängig von der Zusammensetzung des zur Verflüssigung gelangenden Gasgemisches, insbesondere von dessen Stickstoffanteil und von der Verflüssigungstemperatur. Häufig auftretende nicht-verflüssigbare Gemische haben in etwa folgende Zusammensetzung (Angaben in Vol.-%):

Stickstoff 20 bis 40%, Kohlenmonoxid 5 bis 30%, Tetrafluorethylen 30 bis 60%, Kohlendioxid 2 bis 6%, Sauerstoff 0,1 bis 0,3%, Difluormonochlormethan 0,2 bis 2%, Trifluormethan 1,0 bis 4%, sonstige tiefer als Tetrafluorethylen siedende Pyrolyseprodukte 1 bis 2%.

Der vorliegenden Erfindung liegt das Problem zugrunde, die Tetrafluorethylen-Anteile aus solchen nicht-verflüssigbaren Teilströmen der Difluormonochlormethan-Pyrolyseprodukte, aus Spülgasen der Tetrafluorethylen-Herstellung und -Polymerisation oder aus Gemischen derselben von den darin enthaltenen beträchtlichen Anteilen an Stickstoff und/oder Kohlenmonoxid abzutrennen und das Tetrafluorethylen der Wiederverwendung zuzuführen.

Aus der japanischen Patentbekanntmachung Sho 48-10 444 ist ein Verfahren zur Isolierung und Wiedergewinnung von Tetrafluorethylen aus einem Gasgemisch von Tetrafluorethylen und niedriger siedenden Anteilen bekannt, wobei dort

unter niedriger siedenden Anteilen die bei der Tetrafluorethylen-Herstellung durch Pyrolyse von Difluormonochlormethan in Gegenwart von Wasserdampf entstehenden Nebenprodukte verstanden werden, nämlich neben Kohlenmonoxid verschiedene niedrig-siedende fluorhaltige Pyrolyseprodukte wie beispielsweise Trifluormethan, Vinylidenfluorid oder Hexafluorethan. Bei diesem Verfahren wird die zunächst durch Destillation gewonnene Vorlauffraktion, bestehend aus Tetrafluorethylen und den niedriger siedenden Komponenten, unter Anwendung von erheblichem Überdruck mit einem flüssigen Perhalogenkohlenwasserstoff in Berührung gebracht, wobei das Tetrafluorethylen absorbiert und dann durch Entspannen aus dem Perhalogenkohlenwasserstoff wieder in Freiheit gesetzt wird. Der wesentliche Nachteil dieses Verfahrens besteht darin, dass die Absorption des Tetrafluorethylens in dem flüssigen Perhalogenkohlenwasserstoff unter Anwendung von erheblichem Überdruck erfolgt, da die Anwendung eines solchen Überdrucks wegen der oben erwähnten Neigung des Tetrafluorethylens zum explosiven Selbstzerfall mit einem erheblichen Sicherheitsrisiko behaftet ist. Anlagenteile, die unter einem Tetrafluorethylen-Druck von mehr als 6 bar betrieben werden, erfordern einen hohen sicherheitstechnischen Aufwand.

Aus der DE-OS 2638650 ist weiterhin ein Verfahren bekannt, in dem ebenfalls die Abtrennung von Tetrafluorethylen aus Gasgemischen mit Stickstoff und/oder Kohlenmonoxid beschrieben ist. Bei diesem Verfahren wird der aus der Verflüssigung der Pyrolysegase des Difluormonochlormethans anfallende Gasstrom mit einem gesättigten aliphatischen Keton, einem gesättigten aliphatischen Kohlenwasserstoff oder Gemischen solcher Verbindungen mit einem Siedepunkt im Bereich von 50 bis 130°C in Kontakt gebracht, wobei Stickstoff, Kohlenmonoxid oder deren Gemische gasförmig entweichen und das Tetrafluorethylen, gegebenenfalls zusammen mit anderen niedriger als Tetrafluorethylen siedenden Komponenten absorbiert und anschliessend durch Erwärmen des beladenen Absorptionsmittels auf seinen Siedepunkt wieder in Freiheit gesetzt wird, wobei ferner gegebenenfalls anschliessend Tetrafluorethylen von diesen anderen niedriger als Tetrafluorethylen siedenden Komponenten nach bekannten Methoden getrennt wird.

Beiden Verfahren ist gemeinsam, dass das verwendete Absorptionsmittel nach der Beladung mit Tetrafluorethylen von diesem wieder abgetrennt wird, wozu im Anschluss an die Absorptionsvorrichtung eine zusätzliche Desorptionsvorrichtung erforderlich ist, was einen zusätzlichen apparativen und energetischen Aufwand erfordert. Ausserdem ist die Reinigung des gebrauchten Absorptionsmittels von Pyrolyseprodukten, die bei der Desorption des Tetrafluorethylens im Absorptionsmittel verbleiben, nicht einfach zu bewerkstelligen, die Beseitigung des Absorptionsmittels ist andererseits unwirtschaftlich und aus Gründen des Umweltschutzes problematisch.

Die Aufgabe, diese Nachteile zu vermeiden, wird im Rahmen der Erfindung gelöst durch ein Verfahren der eingangs genannten Art, das dadurch gekennzeichnet ist, dass dieses Gasgemisch mit der 5- bis 10fachen Gewichtsmenge, bezogen auf im Gasgemisch anwesendes Tetrafluorethylen, eines flüssigen Absorptionsmittels, das eine Temperatur von −30 bis −100°C aufweist und das aus

a) mindestens einer der Verbindungen Difluormonochlormethan, Tetrafluormonochlorethan ode Hexafluormonochlorpropan, oder

b) einer Fraktion, die bei der fraktionierenden Aufarbeitung des verflüssigten Anteils der Pyrolyseprodukte nach Abtrennung des Tetrafluorethylens gewonnen wird und die wenigstens 40 Gew.-% zumindest einer der unter a) genannten Verbindungen enthält,

besteht, in innigen Kontakt gebracht wird, wobei das Absorptionsmittel, das Tetrafluorethylen aufnimmt und die verbleibenden nicht-verflüssigbaren Komponenten freisetzt, und dass das mit Tetrafluorethylen beladene Absorptionsmittel in die Verflüssigung der Pyrolyseprodukte zurückgeführt wird.

Die Lösung der genannten Aufgabe beinhaltet also im breitesten Sinn dieser Erfindung, dass ein dem Pyrolyseprozess des Difluormonochlormethans oder dessen Aufarbeitungsstufen «arteigener» Stoff als Absorptionsmittel Einsatz findet.

«Arteigener» Stoff im Pyrolyseprozess oder dessen Aufarbeitungsschritten ist in erster Linie das Difluormonochlormethan selbst, das entweder als Ausgangsprodukt oder, wegen der unvollständigen Umsetzung bei der Pyrolyse, im Zuge der Aufarbeitung durch fraktionierte Destillation in praktisch reiner Form wiedergewonnen wird.

Weitere «arteigene» Stoffe im Sinne der vorliegenden Erfindung, wie sie als Absorptionsmittel Einsatz finden, sind auch solche, die bei der fraktionierenden Aufarbeitung des verflüssigten Anteils anfallen. Eine solche fraktionierende Aufarbeitung des verflüssigten Teilstroms der Pyrolysegase, die neben Tetrafluorethylen ein breites Spektrum von Fluorchlorkohlenstoffen, Fluorchlorkohlenwasserstoffen, Fluorkohlenstoffen und Fluorkohlenwasserstoffen enthalten, erfolgt prinzipiell in der Weise, dass dieser Teilstrom in einer Fraktionierkolonne durch fraktionierte Destillation von den niedriger als Tetrafluorethylen siedenden Komponenten befreit wird, wie zum Beispiel Trifluormethan, Difluormethan und Difluorethylen. Die Sumpffraktion dieser Kolonne wird der folgenden Kolonne zugeleitet, in der über Kopf das erwünschte Hauptprodukt Tetrafluorethylen abgenommen wird, während im Sumpf dieser Kolonne alle übrigen, höher als Tetrafluorethylen siedenden Verbindungen verbleiben, wie insbesondere die unumgesetzte Ausgangsverbindung Difluormonochlormethan, ferner Octafluorcyclobutan, Hexafluorpropen, Tetrafluormonochlormethan, Hexafluormonochlorpropan und eine grosse Zahl anderer fluorierter und

chlorfluorierter Kohlenstoff- und Kohlenwasserstoffverbindungen. Diese Verbindungen werden in weiteren Schritten durch fraktionierte Destillation und/oder Extraktivdestillation aufgetrennt, wobei die Zahl und die Zusammensetzung dieser weiteren abgenommenen Fraktionen von den jeweiligen Bedingungen des nach der Abtrennung des Tetrafluorethylens angewandten Fraktionierungsverfahrens abhängt. Ausser der Ausgangsverbindung Difluormonochlormethan werden Tetrafluormonochlormethan und Hexafluormonochlorpropan als im wesentlichen reine Verbindungen zurückgewonnen, während die übrigen, bei der fraktionierten Destillation gewonnenen Fraktionierungsschnitte oder Azeotrope zumindest eine dieser drei genannten Verbindungen zu wenigstens 40 Gew.-%, vorzugsweise zu wenigstens 50 Gew.-%, enthält.

Als weitere Bestandteile enthalten solche durch fraktionierte Destillation des verflüssigten Teilstroms der Pyrolyseprodukte gewonnenen Fraktionen Fluorkohlenstoff-Verbindungen, Fluorkohlenwasserstoff-Verbindungen, Chlorfluorkohlenstoff-Verbindungen oder andere Chlorfluorkohlenwasserstoff-Vebindungen der Summenformel $C_aF_bH_cCl_d$, worin a eine ganze Zahl von 1 bis 6, b eine ganze Zahl von 2 bis 14, c eine ganze Zahl von 0 bis 2 und d eine ganze Zahl von 0 bis 2 bedeutet. Art und Anteil dieser Verbindungen in den Fraktionen können sehr stark schwanken in Abhängigkeit von den Bedingungen des Pyrolyseverfahrens und den Bedingungen der Aufarbeitung des verflüssigten Teilstroms.

Dies sind insbesondere $\alpha$) cyclische oder einfach ungesättigte acyclische Fluorkohlenstoff-Verbindungen der Summenformel $(CF_2)_n$, worin n eine ganze Zahl von 3 bis 5 bedeutet, wie vor allem Hexafluorpropen und Octafluorcyclobutan; ferner $\beta$) Chlorfluorkohlenstoff-Verbindungen der Summenformel $(CF_2)_nCl_2$, worin n eine ganze Zahl von 1 bis 4 bedeutet, wie vor allem Difluormonochlormethan und Trifluortrichlorethan; ferner $\gamma$) Fluorkohlenwasserstoff-Verbindungen der Summenformel $(CF_2)_nHF$, worin n eine ganze Zahl von 3 bis 4 bedeutet, also Heptafluorpropan und Nonafluorbutan; ferner $\delta$) Chlorfluorkohlenstoff-Verbindungen der Summenformel $(CF_2)_nFCl$, worin n eine ganze Zahl von 2 bis 4 bedeutet, also Pentafluormonochlorethan, Heptafluormonochlorpropan und Nonafluormonochlorbutan sowie $\varepsilon$) Octafluormonochlorbutan.

Die besonders bevorzugten Absorptionsmittel im Rahmen des erfindungsgemässen Verfahrens sind das Difluormonochlormethan, das als Ausgangsprodukt der Pyrolyse von aussen oder als im Rahmen der fraktionierten Destillation der Pyrolyseprodukte rein oder nahezu rein gewonnene Verbindung dem Absorptionsvorgang zugeführt werden kann, sowie Fraktionen, die mindestens 70 Gew.-% Hexafluormonochlorpropan und maximal 30 Gew.-% Komponenten der obengenannten Gruppen $\alpha$ bis $\varepsilon$) oder mindestens 50 Gew.-% Tetrafluormonochlorethan und maximal 50 Gew.-% der vorgenannten Komponenten enthalten.

Das Absorptionsmittel, das aus dem Aufarbeitungsprozess im flüssigen Zustand zur Verfügung steht oder in diesen gebracht werden muss, wird (je nach seinem Siedepunkt, der mindestens etwa 5°C oberhalb der Behandlungstemperatur liegen soll) bei Temperaturen von -30 bis -100°C, vorzugsweise -40 bis -80°C, vorzugsweise im Gegenstrom, in einer Absorptionskolonne mit dem Gasgemisch in innigen Kontakt gebracht. Die Absorption findet bei praktisch drucklosen Bedingungen (1 bar bis gegebenenfalls 1,5 bar) statt.

Als Absorptionskolonnen verwendet man mit Vorteil Glockenbodenkolonnen oder Füllkörperkolonnen. Die je nach apparativen Gegebenheiten anzuwendenden Gasgeschwindigkeiten liegen im üblichen Bereich von Stoffaustauschvorgängen und sind für die Absorption nicht kritisch.

Die Menge an Absorptionsmittel, die mit dem Gasgemisch in Kontakt gebracht wird, liegt bei der 5- bis 10fachen, vorzugsweise der 6- bis 8fachen Gewichtsmenge, bezogen auf das im Gasgemisch anwesende Tetrafluorethylen.

Unter diesen Bedingungen nimmt das Absorptionsmittel im wesentlichen das Tetrafluorethylen und das gegebenenfalls anwesende Difluormonochlormethan auf und setzt Stickstoff und/oder Kohlenmonoxid sowie die gegebenenfalls anderen, unter den Bedingungen der Verflüssigung der Difluormonochlormethan-Pyrolyseprodukte nicht-verflüssigbaren Komponenten in Freiheit. Lediglich kleine Mengen an Tetrafluorethylen und Difluormonochlormethan, entsprechend dem Partialdruck über der Lösung im Absorptionsmittel, verbleiben im Abgasstrom.

Da die Menge an Absorptionsmittel im Verhältnis zum gesamten verflüssigten Anteil der Pyrolysegase relativ klein ist (1:15 bis 1:12), ergeben sich bei der Zurückleitung des Absorptionsmittels zusammen mit dem absorbierten Tetrafluorethylen indas Aufarbeitungsverfahren derPyrolyseprodukte keine Probleme, vorzugsweise erfolgt die Zurückleitung in den bereits verflüssigten Teilstrom des Aufarbeitungsverfahrens.

Das von Tetrafluorethylen nahezu vollständig befreite Abgas kann nun beim Entweichen aus der Absorptionskolonne problemlos beseitigt werden.

Die Vorteile des erfindungsgemässen Verfahrens liegen in der Benutzung eines dem Verfahren «arteigenen» Stoffes als Absorptionsmittel begründet. Es brauchen weder kostspielige «artfremde» Absorptionsmittel beschafft noch brauchen solche nach dem Absorptionsvorgang abgetrennt und wiedergewonnen zu werden. Da das Absorptionsmittel zusammen mit dem abgetrennten und somit aus dem verflüssigten Abgasstrom zurückgewonnenen Tetrafluorethylen in den Aufarbeitungsprozess zurückgeleitet wird, entfällt ferner jeder energetische und apparative Aufwand für die Desorption von Tetrafluorethylen aus dem Absorptionsmittel.

Die Erfindung wird durch folgende Beispiele erläutert.

Die Versuche wurden in einer Glockenbodenkolonne mit 80 mm Durchmesser mit 11 Glocken

bei 120 mm Bodenabstand durchgeführt.

Als Absorptionsmittel wurden die jeweils angegebenen reinen Komponenten beziehungsweise Gemische eingesetzt. Das Absorptionsmittel mit dem absorbierten Tetrafluorethylen wurde in den verflüssigten Teilstrom zurückgeleitet.

Erklärung der Abkürzungen:

F 22 = Difluormonochlormethan
F 23 = Trifluormethan
F 124 = Tetrafluormonochlorethan
F 226 = Hexafluormonochlorpropan
HFP = Hexafluorpropen
$C_4F_8$ = Octafluorcyclobutan

«Sonstige Komponenten», die nicht einzeln identifiziert wurden, sind solche der obengenannten Summenformel $C_aF_bH_cCl_d$ mit den dort gegebenen Definitionen.

Beispiel 1
Absorptionsmittel: Difluormonochlormethan
Menge des Absorptionsmittels: 64 kg/h
Temperatur des Absorptionsmittels: -60°C

| Stoff | Abgasstrom vor der Absorption Vol.-% | m³/h | kg/h | nach der Absorption Vol.-% | m³/h | kg/h |
|---|---|---|---|---|---|---|
| $N_2$ | 34,2 | 1,37 | 1,71 | 43,66 | 1,37 | 1,71 |
| CO | 12,7 | 0,51 | 0,63 | 16,22 | 0,51 | 0,64 |
| $CO_2$ | 4,1 | 0,16 | 0,32 | – | – | – |
| TFE | 45,5 | 1,82 | 8,12 | 0,20 | <0,01 | 0,02 |
| F 22 | 0,8 | 0,03 | 0,12 | 38,02 | 1,19 | 4,60 |
| F 23 und sonstige Komponenten | 2,7 | 0,11 | 0,32 | 1,90 | 0,06 | 0,18 |
| Gesamt | | 4,0 | | | 3,13 | |

Beispiel 2
Absorptionsmittel: Difluormonochlormethan
Menge des Absorptionsmittels: 60 kg/h
Temperatur des Absorptionsmittels: –72°C

| Stoff | Abgasstrom vor der Absorption Vol.-% | m³/h | kg/h | nach der Absorption Vol.-% | m³/h | kg/h |
|---|---|---|---|---|---|---|
| $N_2$ | 35,0 | 1,56 | 1,95 | 61,18 | 1,56 | 1,95 |
| CO | 9,3 | 0,41 | 0,51 | 16,08 | 0,41 | 0,51 |
| $CO_2$ | 3,8 | 0,17 | 0,33 | – | – | – |
| TFE | 48,7 | 2,17 | 9,68 | 0,16 | <0,01 | 0,01 |
| F 22 | 0,7 | 0,03 | 0,12 | 20,78 | 0,53 | 2,05 |
| F 23 und sonstige Komponenten | 2,5 | 0,11 | 0,34 | 1,96 | 0,05 | 0,16 |
| Gesamt | | 4,45 | | | 2,55 | |

Beispiel 3
Absorptionsmittel: Tetrafluormonochlorethan
Menge des Absorptionsmittels: 58 kg/h
Temperatur des Absorptionsmittels: –66°C

| Stoff | Abgasstrom vor der Absorption Vol.-% | m³/h | kg/h | nach der Absorption Vol.-% | m³/h | kg/h |
|---|---|---|---|---|---|---|
| $N_2$ | 35,4 | 1,63 | 2,04 | 69,97 | 1,63 | 2,04 |
| CO | 10,4 | 0,48 | 0,60 | 20,62 | 0,48 | 0,60 |
| $CO_2$ | 4,2 | 0,19 | 0,37 | – | – | – |
| TFE | 46,4 | 2,15 | 9,59 | 0,16 | <0,01 | 0,01 |
| F 22 | 0,8 | 0,04 | 0,14 | 0,18 | <0,01 | 0,01 |
| F 23 und sonstige Komponenten | 2,8 | 0,13 | 0,39 | 2,57 | 0,06 | 0,18 |
| F 124 | – | – | – | 6,00 | 0,14 | 0,85 |
| Gesamt | | 4,62 | | | 2,33 | |

Beispiel 4
Absorptionsmittel: Hexafluormonochlorpropan
Menge des Absorptionsmittels: 58 kg/h
Temperatur des Absorptionsmittels: -68°C

| Stoff | Abgasstrom vor der Absorption Vol.-% | m³/h | kg/h | nach der Absorption Vol.-% | m³/h | kg/h |
|---|---|---|---|---|---|---|
| $N_2$ | 36,4 | 1,65 | 2,06 | 76,53 | 1,65 | 2,06 |
| CO | 9,3 | 0,42 | 0,53 | 19,53 | 0,42 | 0,53 |
| $CO_2$ | 3,6 | 0,16 | 0,31 | - | - | - |
| TFE | 47,7 | 2,17 | 9,68 | 0,10 | <0,01 | 0,01 |
| F 22 | 0,7 | 0,03 | 0,12 | 0,12 | <0,01 | 0,01 |
| F 23 | 2,3 | 0,11 | 0,34 | 2,79 | 0,06 | 0,18 |
| und sonstige Komponenten | | | | | | |
| F 226 | | | | 0,93 | 0,02 | 0,17 |
| Gesamt | | 4,54 | | | 2,17 | |

Beispiel 5
Absorptionsmittel:

Gemisch aus Tetrafluormonochlorethan (60 Gew.-%) und Octafluorcyclobutan (40 Gew.-%)
Menge des Absorptionsmittels: 60 kg/h
Temperatur des Absorptionsmittels: -65°C

| Stoff | Abgasstrom vor der Absorption Vol.-% | m³/h | kg/h | nach der Absorption Vol.-% | m³/h | kg/h |
|---|---|---|---|---|---|---|
| $N_2$ | 37,9 | 1,84 | 2,30 | 74,5 | 1,84 | 2,30 |
| CO | 7,8 | 0,38 | 0,47 | 15,4 | 0,38 | 0,47 |
| $CO_2$ | 4,0 | 0,19 | 0,37 | - | - | - |
| TFE | 46,3 | 2,25 | 10,03 | - | <0,01 | 0,01 |
| F 22 | 0,9 | 0,04 | 0,15 | - | <0,01 | 0,01 |
| F 23 | 3,1 | 0,15 | 0,47 | 3,24 | 0,08 | 0,25 |
| und sonstige Komponenten | | | | | | |
| F 124 | | | | 4,45 | 0,11 | 0,67 |
| $C_4F_8$ | | | | 2,42 | 0,06 | 0,57 |
| Gesamt | | 4,86 | | | 2,47 | |

Beispiel 6
Absorptionsmittel:

Gemisch aus Difluormonochlormethan (89,5 Gew.-%) und Hexafluorpropen (10,5 Gew.-%)
Menge des Absorptionsmittels: 62 kg/h
Temperatur des Absorptionsmittels: -70°C

| Stoff | Abgasstrom vor der Absorption Vol.-% | m³/h | kg/h | nach der Absorption Vol.-% | m³/h | kg/h |
|---|---|---|---|---|---|---|
| $N_2$ | 36,8 | 1,69 | 2,11 | 62,4 | 1,69 | 2,11 |
| CO | 8,2 | 0,38 | 0,47 | 14,0 | 0,38 | 0,47 |
| $CO_2$ | 3,8 | 0,17 | 0,33 | - | - | - |
| TFE | 47,8 | 2,20 | 9,81 | 0,1 | < 0,01 | 0,01 |
| F 22 | 0,8 | 0,04 | 0,15 | 20,2 | 0,55 | 2,08 |
| F 23 | 2,6 | 0,12 | 0,37 | 1,8 | 0,05 | 0,16 |
| und sonstige Komponenten | | | | | | |
| HFP | | | | 1,5 | 0,04 | 0,25 |
| Gesamt | | 4,60 | | | 2,72 | |

Beispiel 7
Absorptionsmittel:
Gemisch aus Hexafluormonochlorpropan (70 Gew.-%) Octafluormonochlorbutan (25 Gew.-%) und Nebenkomponenten +) (05 Gew.-%)
Menge des Absorptionsmittels: 60 kg/h
Temperatur des Absorptionsmittels: –70°C

| Stoff | Abgasstrom vor der Absorption Vol.-% | $m^3/h$ | kg/h | nach der Absorption Vol.-% | $m^3/h$ | kg/h |
|---|---|---|---|---|---|---|
| $N_2$ | 36,2 | 1,88 | 2,35 | 75,5 | 1,88 | 2,35 |
| CO | 9,8 | 0,51 | 0,64 | 20,48 | 0,51 | 0,64 |
| $CO_2$ | 3,7 | 0,19 | 0,37 | – | – | – |
| TFE | 46,5 | 2,42 | 10,80 | 0,08 | 0,002 | 0,03 |
| F 22 | 0,9 | 0,05 | 0,20 | 0,12 | 0,003 | 0,01 |
| F 23 und sonstige Komponenten | 2,9 | 0,15 | 0,47 | 2,57 | 0,064 | 0,20 |
| F 226 | | | | 1,0 | 0,025 | 0,20 |
| Octafluormonochlorbutan | | | | 0,16 | 0,004 | 0,04 |
| Gesamt | | 5,2 | | | 2,49 | |

* Nebenkomponenten sind Verbindungen mit der oben definierten Summenformel $C_aF_bH_cCl_d$, die einen Siedepunkt nahe dem des F 226 und Octafluormonochlorbutans besitzen.

**Patentansprüche**

1. Verfahren zur Abtrennung von Tetrafluorethylen aus einem Gasgemisch, das bei der Verflüssigung der Pyrolyseprodukte des Difluormonochlormethans bei Drücken von 1 bis 5 bar als nicht-verflüssigbarer Anteil, bei der Tetrafluorethylen-Herstellung oder -Polymerisation als Spülgas anfällt oder durch Vermischen solcher Gase gebildet wird und das neben Tetrafluorethylen Kohlenmonoxid, Stickstoff oder Gemische dieser beiden Gase, ferner gegebenenfalls kleinere Anteile von Difluormonochlormethan sowie von dessen nicht-verflüssigbaren Pyrolyseprodukten enthält, dadurch gekennzeichnet, dass dieses Gasgemisch mit der 5- bis 10fachen Gewichtsmenge, bezogen auf im Gasgemisch anwesendes Tetrafluorethylen, eines flüssigen Absorptionsmittels, das eine Temperatur von –30 bis –100°C aufweist und das aus

a) mindestens einer der Verbindungen Difluormonochlormethan, Tetrafluormonochlorethan oder Hexafluormonochlorpropan, oder

b) einer Fraktion, die bei der fraktionierenden Aufarbeitung des verflüssigten Anteils der Pyrolyseprodukte nach Abtrennung des Tetrafluorethylens gewonnen wird und die wenigstens 40 Gew.-% zumindest einer der unter a) genannten Verbindungen enthält,

besteht, in innigen Kontakt gebraucht wird, wobei das Absorptionsmittel des Tetrafluorethylen aufnimmt und die verbleibenden nicht-verflüssigbaren Komponenten freisetzt, und dass das mit Tetrafluorethylen beladene Absorptionsmittel in die Verflüssigung der Pyrolyseprodukte zurückgeführt wird.

2. Verfahren nach Anspruch 1, dadurch gekennzeichnet, dass die als Absorptionsmittel eingesetzte Fraktion neben den Anteilen der unter a) genannten Verbindungen eine Fluorkohlenstoff-Verbindung, Fluorkohlenwasserstoff-Verbindung, Chlorfluorkohlenstoff-Verbindung oder Chlorfluorkohlenwasserstoff-Verbindung der Summenformel

$C_aF_bH_cCl_d$,

worin a eine ganze Zahl von 1 bis 6, b eine ganze Zahl von 2 bis 14, c eine ganze Zahl von 0 bis 2 und d eine ganze Zahl von 0 bis 2 bedeutet, enthält.

**Claims:**

1. A process for separating tetrafluoroethylene from a gaseous mixture obtained in the tetrafluoroethylene liquefaction step of the pyrolysis products of difluoromonochloromethane and from scavenging gases of tetrafluoroethylene manufacture and polymerization processes or by mixing such gases and containing, in addition to tetrafluoroethylene, nitrogen, carbon monoxide or mixtures thereof and optionally minor amounts of difluorochloromethane and its non-liquefiable pyrolysis products, characterised by contacting said gaseous mixture with a liquid absorption medium being present in an amount of from 5 to 10 parts by weight per part by weight of tetrafluoroethylene in said gaseous mixture and having a temperature of from –30°C to –100°C and comprising

a) difluoromonochloromethane, tetrafluoromonochloroethane, hexafluoromonochloropropane, and mixtures thereof, and

b) a fraction obtained in the fractional distillation process of the pyrolysis products of difluoromonochloromethane after liquefying and separating the liquefied tetrafluoroethylene, said fraction containing at least 40% by weight of group a) components, whereby absorbing the tetrafluoroethylene and liberating the remaining non-liquefiable components, and then recycling the absorption medium charged with tetrafluoroethylene into the liquefied portion of said pyrolysis products.

2. The process of claim 1, characterised in that the fraction used as absorption medium contains in addition to the group a) components a fluorocarbon, fluorohydrocarbon, chlorofluorocarbon, or chlorofluorohydrocarbon compound of the general formula

$C_aF_bH_cCl_d$

in which a is an integer of from 1 to 6, b is an integer of from 2 to 14, c is an integer of from 1 to 2 or is zero, and d is an integer of from 1 to 2 or is zero.

## Revendications

1. Procédé pour séparer le tétrafluoréthylène d'un mélange gazeux qui est engendré sous la forme d'une fraction non-liquéfiable lors de la liquéfaction des produits de la pyrolyse du difluoromonochlorométhane sous une pression de 1 à 5 bars, ou qui est présent comme gaz de balayage dans la fabrication ou la polymérisation du tétrafluoréthylène, ou qui est formé par mélange de tels gaz, ce mélange gazeux contenant, en plus du tétrafluoréthylène, du monoxyde de carbone, de l'azote ou un mélange de ces deux gaz, ainsi qu'éventuellement de plus faibles quantités de difluoromonochlorométhane et de produits non-liquéfiables de la pyrolyse de ce dernier, caractérisé en ce que l'on place ledit mélange gazeux en contact intime avec une quantité de 5 à 10 fois supérieure en poids, rapportée au tétrafluoréthylène présent dans le mélange gazeux, d'un agent d'absorption liquide qui présente une température de -30 à -100°C et qui se compose de:

a) Au moins un composé choisi parmi le difluoromonochlorométhane, le tétrafluoromonochloréthane et l'hexafluoromonochloropropane, ou:

b) Une fraction qui est obtenue, après séparation du tétrafluoréthylène, lors du fractionnement de la partie liquéfiée des produits de pyrolyse et qui contient au moins 40% en poids de l'un au moins des composés spécifiés en a),

l'agent d'absorption absorbant le tétrafluoréthylène et libérant les constituants non-liquéfiables restants, et en ce que l'agent d'absorption chargé de tétrafluoréthylène est recyclé vers la liquéfaction des produits de pyrolyse.

2. Procédé selon la revendication 1, caractérisé en ce que la fraction introduite comme agent d'absorption contient, en plus des quantités des composés spécifiés en a), un composé fluorocarboné, un composé hydrogéno-fluorocarboné, un composé chloro-fluorocarboné ou un composé hydrogénochloro-fluorocarboné de formule générale:

$C_aF_bH_cCl_d$,

oú a est un entier de 1 à 6, b un entier de 2 à 14, c un entier de 0 à 2 et d un entier de 0 à 2.